# EUROPEAN PATENT SPECIFICATION

(11) **EP 1 605 776 B1**
(45) Date of publication and mention of the grant of the patent: **09.05.2012**
(21) Application number: 04716471.0
(22) Date of filing: 02.03.2004
(51) Int. Cl.: A23L 1/054, A23K 1/18, A61K 47/36

(54) **METHOD AND COMPOSITION FOR PREVENTING OR REDUCING DIARRHEA**
VERFAHREN UND ZUSAMMENSETZUNG ZUR PRÄVENTION ODER VERRINGERUNG VON DIARRHÖE
METHODE ET COMPOSITION PERMETTANT DE PREVENIR OU DE REDUIRE LA DIARRHEE

(30) Priority: 12.03.2003 US 387167
(43) Date of publication of application: 21.12.2005
(73) Proprietor: Hill's Pet Nutrition, Topeka, KS 66603 (US)
(72) Inventor: CHEUK, Wai, San Marcos, California 92078 (US); KHOO, Christina, Lawrence, Kansas 66047 (US)
(74) Representative: Jenkins, Peter David
(86) International application number: PCT/US2004/006358
(87) International publication number: WO 2004/080206

(56) References cited:
- GB-A- 2 385 330
- ANDERSON D M W ET AL: "THE DIETARY EFFECTS OF GELLAN GUM IN HUMANS" FOOD ADDITIVES AND CONTAMINANTS, vol. 5, no. 3, 1988, pages 237-250, XP009033726 ISSN: 0265-203X

## Description

### BACKGROUND OF THE INVENTION

All mammals require a healthy diet and proper digestion of that diet for continued growth and ordinary well being. However, gastrointestinal distress interferes with the ordinary digestion of food. Some of these problems can be quite serious and demand serious medical attention such as Crohn's disease; irritable bowel syndrome, other chronic conditions and the like. Others are of a less serious condition and can be essentially self-limiting such as food borne virus, intestinal flu and the like. Almost all of them are accompanied by diarrhea, a loose watery stool which can be extremely unpleasant to the mammal harboring the condition or to a pet owner who must clean up after the pet evacuates, particularly if on a chronic basis.

It has been found that some pet foods tend to create or exacerbate a diarrhea condition, see for example USP 6,280,779 issued August 28, 2001. Particularly with pets eating a "chunk and gravy" diet, diarrhea can be a significant problem. The presence of significant quantities of gum in the diet, primarily chemically modified starches or gums, but even, to a lesser extent, ordinary natural starches and gums has been associated with this issue.

It has now been found that a specific gum not only does not have the tendency to create or exacerbate a diarrhea condition in a mammal, but has the ability to prevent or reduce such diarrhea in a mammal.

### SUMMARY OF INVENTION

In accordance with the invention, there is a composition comprising gellan gum for use in preventing diarrhea in a mammal that has a tendency to have diarrhea or reducing diarrhea in a mammal already experiencing diarrhea.

Further, there is a pharmaceutical unit dosage form suited for oral administration to a mammal comprising a daily dosage or portion of a daily dosage of gellan gum sufficient to prevent diarrhea or reduce diarrhea in a mammal, in association with a pharmaceutical carrier.

Additionally, there is a wet food composition suitable for ingestion by a dog or cat and having at least one food component and a diarrhea preventing or diarrhea reducing amount of gellan gum.

### DETAILED DESCRIPTION OF THE INVENTION

"Chunks and gravy" diet as aforementioned includes diets wherein the chunks are pieces of meat or meat by-products. This is the primary content of the chunk. Also present in the chunk are usually grains and fibrous materials as well as vitamins and nutrients. These materials are generally present as the minor portion of the chunk. The gravy portion usually has a fluid characteristic and supplies aroma, palatability, and some additional nutritional properties to the fond product such as additional vitamins, minerals, and the like. Also present in the market place are other discrete meaty forms in a discrete meat portion in somewhat elongated, as relatively flat as in a delicatessen sliced meat. As utilized throughout the specification and claims the terms "chunk" shall include slices as well as any other discrete meat containing composition which is separate from the discrete gravy component of the diet. In each of these cases, the "chunks" are present with the gravy as a single unit, for example, sold in a container.

It has now been found that the use of a specific gum, gellan, substantially reduces diarrhea when a mammal defecates. Gellan gum can prevent diarrhea in a mammal specifically in a mammal that has a tendency to have diarrhea from time to time. Gellan gum also has the ability to reduce diarrhea in a mammal already experiencing diarrhea. This is a reduction which can go from simple visual observation of reduction to a statistically significant reduction to a virtual elimination or rollback of the diarrhea state. This can occur through almost all etiologies which are at least partially characterized by a state of diarrhea. However, it is preferred that the etiology be unknown or induced through a bacterial, viral (both preferably short term) or a dietary regimen.

The gellan gum can be administered to the mammal, preferably one in need of such administration in any one of many ways, such as oral, rectal, and the like, although oral is definitely preferred. The gellan gum can be administered in a wet diet, either incorporated therein or on the surface of any diet component, such as, by spraying or precipitation thereon. It can be present in the nutritional diet per se or in a snack or a treat. It can also be present in the liquid portion of the diet such as water or another fluid. The gellan can be administered as a powder solid or as a liquid such as a gel. If desired the gellan gum can be orally administered in a pharmaceutical dosage form such as a capsule, tablet, caplet, syringe, and the like. Within the dosage form the gellan gum can be present as a powder or a liquid such as a gel. Any of the usual pharmaceutical carriers can be employed such as water, glucose, sucrose and the like together with the gellan gum.

With respect to administering to a dog or cat, the daily dosage minimum is at least about 0.1% by weight of food or at least about 0.05 g/kg bwt, preferably at least about 0.2% by weight, most preferably at least about 0.5% by weight or at least about 0.3 g/kg bwt. The maximum amount is below that which can bring about significant undesirable side effects. Generally no more than about 1.5 or 2% by weight of food and no more than about 4% by weight of the food or 1 or 2 g/kg bwt can be employed. A minimum dosage for administering to a human is about 0.05 g/kg bwt or preferably at least about 0.1 g/kg bwt. The maximum amount is below that which can bring about significant undesirable side effects. Generally no more than about 1 or 2 g/kg bwt or about 4% by weight of the food can be employed.

Most interestingly when the cause of the diarrhea seems to be a food component, the component need not be removed completely or to any great extent from the diet for the gellan gum to be effective in combating the diarrhea.

As used in this specification and claims, the term "gellan gum" refers to a linear polysaccharide made from fermentation by Sphingomonas paucimobilis (elodea) (ATCC31461). Industrial preparation of the gum can be carried out by inoculating Sphingomonas paucimobilis into a fermentation broth containing glucose, glucuronic acid and rhamnose to form a tetrasaccharide repeating unit in a ratio of 2:1:1. In its native form, it is highly acylated with 1.5 acylgroup, acetyl and glycerate, per repeating unit. Modifications of the acyl groups both in number and type can be made as long as the basic anti diarrhea activity of the gellan gum is not significantly diminished. These different forms can be obtained from CP Kelco under different tradenames including Gelrite®, K9A50 and other Kelco gellan gums including but not limited to, Kelcogel LT®, Kelcogel F, and Kelcogel LT100®. As used throughout the specifications "gellan" refers to the natural gum or acyl modified gum as long as the anti-diarrhea function is maintained.

Below are examples of the invention indicating the broad scope and nature of the invention. Wherein numbers 1-5 are used to indicate the physical nature of the stool, these numbers conform to the following observed physical condition of the fecal matter.

Grade 1 Greater than two-thirds of the feces in the defecation are liquid. The feces have lost all form, appearing as a puddle or squirt.

Grade 2 Solid-liquid feces are an intermediate between soft and liquid feces. Approximately equal amounts of feces in defecation are soft and liquid.

Grade 3 Greater than two-thirds of the feces in a defecation are soft. The feces retain enough form to pile but have lost their firm cylindrical appearance.

Grade 4 Firm-soft feces are an intermediate between the grades of firm and soft. Approximately equal amounts of feces in a defecation are firm and soft.

Grade 5 Greater than two-thirds of the feces in a defecation are firm. They have a cylindrical shape with little flattening.

Grades 1 and 2 are unacceptable while grades 4 and 5 are preferred.

### EXAMPLE I: FELINE

Twelve adult domestic shorthair cats, eight males, four females with chronic diarrhea were placed on canned control and test diet which were similar in composition except for the substitution of the 0.2% Kelgum with 1% gellan gum blend (37% gellan gum, 33% sucrose, 18% calcium lactate) or 0.4% pure gellan gum. During the study the cats which were normally on prednisone were weaned off the prednisone 5 days prior to starting the study. The composition of the diet is as show in table 1:

**TABLE 1**

| | Control | Test |
|---|---|---|
| Nutrient | % of diet | % of diet |
| Protein | 8.24 | 7.94 |
| Fat | 9.58 | 9.85 |
| Crude Fiber | 0.3 | 0.2 |
| Moisture | 72.7 | 72.6 |
| Ash | 2.05 | 2.11 |
| Calcium | 0.5 | 0.5 |
| Magnesium | 0.024 | 0.024 |
| Phosphorus | 0.28 | 0.26 |
| Phosphorus | 0.28 | 0.26 |
| Potassium | 0.22 | 0.24 |
| Sodium | 0.073 | 0.094 |
| Chloride | 0.22 | 0.23 |

In a double crossover design, 6 cats were fed control and 6 cats were fed test diet for 14 days. This was followed by a washout period of 14 days when they were all placed on similar washout diets. The cats were then crossed over to either the control or test diet for another 14 days. Stools were graded everyday during the study. A scale of 1-5 was used with 1 being liquid watery diarrhea and 5 being formed and hard stools. The results showed that there was a significant decrease in the frequency of stool scores 1 and 2 with consumption of the test diet (4%) compared to control diet (23%). There was a large increase in the frequency of stool scores of 4 and 5 (49% vs. 27%) in the test compared to the control group. Prior to the beginning the test diet, the average stool score was 2.7. With the test diet, the average stool score was 3.9.

### EXAMPLE 2: DOSE RESPONSE FOR FELINE

12 cats with chronic diarrhea were used in the study, 6 cats were each fed test diets or control diets for 14 days. The tests were repeated with different doses at 0, 0.2, 0.3 and 0.4% gellan gum in the formula. Table 2 shows the percent occurrence of the different types of stool within each diet.

**TABLE II**

| | | Stool Rating | | | |
|---|---|---|---|---|---|
| Dose of gellan (% formula) | 1 | 2 | 3 | 4 | 5 |
| 0.4 | 1 | 2 | 25 | 39 | 33 |
| 0.3 | 0 | 18 | 34.5 | 26 | 18 |
| 0.2 | 1 | 17 | 51 | 18 | 12 |
| 0.1 | 0 | 13 | 48 | 19 | 20 |
| Control | 13 | 16 | 47 | 18 | 1 |

The results showed that gellan gum at a concentration of 0.4% of the diet was able to prevent the incidence of diarrhea (stools 1-2) while at a concentration of 0.1 to 0.3% of the diet, gellan gum was able to reduce the incidence of diarrhea by 2 fold compared to control.

### EXAMPLE 3: CANINE

Dogs fed a grocery brand chunks and gravy formula have been shown to have a significant incidence of diarrhea. This model was used to test the efficacy of gellan gum in reducing the occurrence of diarrhea in dogs. Dogs were fed the grocery brand chunks and gravy formula for 7 days in the control group. In the test group, the dogs were fed the same chunks and gravy formula with 0.4% gellan gum added to the food in the form of a gel. Stool rating were obtained for 7 days. The result in table 3 showed that adding gellan gum to the diet prevented the occurrence of diarrhea in this model.

**TABLE III**

| | | Stool Rating | | | |
|---|---|---|---|---|---|
| | 1 | 2 | 3 | 4 | 5 |
| Control | 24 | 30 | 24 | 18 | 5 |
| Control with 0.4% | 0 | 0 | 18 | 48 | 35 |

### EXAMPLE 4: DOSE RESPONSE FOR CANINE

Using a different chunks and gravy formula for the model of canine diarrhea, gellan gum was incorporated in the gravy formula at different levels. The gravy was formulated with 1% guar gum for the control and test diets. The results are shown below:

**TABLE IV**

| | | Stool Rating | | | |
|---|---|---|---|---|---|
| | 1 | 2 | 3 | 4 | 5 |
| Control | 18 | 9 | 16 | 26 | 31 |
| 0.045% gellan gum | 11 | 9 | 16 | 28 | 36 |
| 0.1% gellan gum | 0 | 10 | 15 | 21 | 54 |
| 0.2% gellan gum | 2 | 11 | 13 | 23 | 51 |
| 0.4% gellan gum | 0 | 8 | 9 | 13 | 70 |

The results showed that 0.1 % gellan gum was able to decrease the incidence of diarrhea to 10% occurrence rate compared to 27% in control. There were little to no incidence of a stool rating of 1 (watery diarrhea).

## Claims

1. A composition comprising gellan gum for use in preventing diarrhea in a mammal that has a tendency to have diarrhea or reducing diarrhea in a mammal already experiencing diarrhea.

2. The composition in accordance with claim 1 wherein the mammal is a human.

3. The composition in accordance with claim 2 wherein the gellan gum is administered orally.

4. The composition in accordance with claim 3 wherein the gellan gum is administered in the form of a liquid or solid.

5. The composition in accordance with claim 4 wherein the gellan gum is administered as a solid in the form of a capsule, tablet or caplet.

6. The composition in accordance with claim 4 wherein the gellan gum is administered as a gel.

7. The composition in accordance with claim 3 wherein the gellan gum is administered in the diet of a human.

8. The composition in accordance with claim 7 wherein the gellan gum is incorporated within the diet or is present on the surface of a diet component.

9. The composition in accordance with claim 3 wherein the gellan gum is administered at a rate of about 0.05 g/kg bwt to about 2 g/kg bwt per day.

10. The composition in accordance with claim 1 wherein the mammal is a dog or cat.

11. The composition in accordance with claim 10 wherein the gellan gum is administered orally.

12. The composition in accordance with claim 11 wherein the gellan gum is administered in the form of a liquid or solid.

13. The composition in accordance with claim 12 wherein the gellan gum is administered as a solid in the form of a capsule, tablet or caplet.

14. The composition in accordance with claim 12 wherein the gellan gum is administered as a gel.

15. The composition in accordance with claim 11 wherein the gellan gum is administered in the diet of the dog or cat.

16. The composition in accordance with claim 15 wherein the gellan gum is incorporated within the diet or is present on the surface of a diet component.

17. The composition in accordance with claim 11 wherein the gellan gum is administered at a rate of about 0.05 g/kg bwt to about 2 g/kg bwt per day.

18. A composition according to any preceding claim which is a pharmaceutical unit dosage form suitable for oral administration to a mammal comprising a daily dosage or portion of a daily dosage of gellan gum sufficient to prevent diarrhea in a mammal that has a tendency to have diarrhea or reduce diarrhea in a mammal in association with a pharmaceutical carrier.

19. A composition as defined in any preceding claim which is a food composition suitable for ingestion by a dog or cat and having at least one food component and a diarrhea preventing or diarrhea reducing amount of gellan gum.

20. The composition in accordance with claim 19 wherein the food selected from a daily diet sufficient to meet the nutritional needs of a dog or cat or treat, or a snack.

21. The composition in accordance with claim 20 wherein the gellan gum is on the surface of a component of the composition.

22. The composition in accordance with claim 19 wherein the gellan gum is from about 0.1 to about 4 wt % of the composition.

23. The composition in accordance with claim 2 whereby the gellan gum is administered to a human in need of the gellan anti-diarrhea effects.

24. The composition in accordance with claim 10 whereby the gellan gum is administered to a dog or cat in need of the gellan anti-diarrhea effects.

## Patentansprüche

1. Zusammensetzung, die Gellan umfasst, zur Verwendung bei der Verhinderung von Durchfall bei einem Säugetier, das eine Neigung zu Durchfall hat, oder zur Verringerung von Durchfall bei einem Säugetier, das bereits Durchfall hat.

2. Zusammensetzung nach Anspruch 1, wobei das Säugetier ein Mensch ist.

3. Zusammensetzung nach Anspruch 2, wobei das Gellan oral verabreicht wird.

4. Zusammensetzung nach Anspruch 3, wobei das Gellan in Form einer Flüssigkeit oder eines Feststoffs verabreicht wird.

5. Zusammensetzung nach Anspruch 4, wobei das Gellan als Feststoff in Form einer Kapsel, Tablette oder kapselförmigen Tablette verabreicht wird.

6. Zusammensetzung nach Anspruch 4, wobei das Gellan als Gel verabreicht wird.

7. Zusammensetzung nach Anspruch 3, wobei das Gellan in der Nahrung eines Menschen verabreicht wird.

8. Zusammensetzung nach Anspruch 7, wobei das Gellan in die Nahrung eingeschlossen ist oder auf der Oberfläche einer Nahrungskomponente vorhanden ist.

9. Zusammensetzung nach Anspruch 3, wobei das Gellan in einer Menge von etwa 0,05 g/kg Körpergewicht bis etwa 2 g/kg Körpergewicht pro Tag verabreicht wird.

10. Zusammensetzung nach Anspruch 1, wobei das Säugetier ein Hund oder eine Katze ist.

11. Zusammensetzung nach Anspruch 10, wobei das Gellan oral verabreicht wird.

12. Zusammensetzung nach Anspruch 11, wobei das Gellan in Form einer Flüssigkeit oder eines Feststoffs verabreicht wird.

13. Zusammensetzung nach Anspruch 12, wobei das Gellan als Feststoff in Form einer Kapsel, Tablette oder kapselförmigen Tablette verabreicht wird.

14. Zusammensetzung nach Anspruch 12, wobei das Gellan als Gel verabreicht wird.

15. Zusammensetzung nach Anspruch 11, wobei das Gellan in der Nahrung des Hundes oder der Katze verabreicht wird.

16. Zusammensetzung nach Anspruch 15, wobei das Gellan in die Nahrung eingeschlossen ist oder auf der Oberfläche einer Nahrungskomponente vorhanden ist.

17. Zusammensetzung nach Anspruch 11, wobei das Gellan in einer Menge von etwa 0,05 g/kg Körpergewicht bis etwa 2 g/kg Körpergewicht pro Tag verabreicht wird.

18. Zusammensetzung nach einem der vorhergehenden Ansprüche, die eine pharmazeutische Dosierform ist, die zur oralen Verabreichung an ein Säugetier geeignet ist, und die eine tägliche Dosis oder Teil einer täglichen Dosis an Gellan, die ausreicht, um Durchfall bei einem Säugetier zu verhindern, das zu Durchfall neigt, oder Durchfall bei einem Säugetier verringert, zusammen mit einem pharmazeutischen Träger umfasst.

19. Zusammensetzung nach einem der vorhergehenden Ansprüche, die eine Futterzusammensetzung ist, die zur Aufnahme durch einen Hund oder eine Katze geeignet ist und die mindestens eine Futterkomponente und eine Durchfall verhindernde oder Durchfall verringernde Menge an Gellan hat.

20. Zusammensetzung nach Anspruch 19, wobei das Futter ausgewählt ist aus einer täglichen Nahrung, die ausreicht, um den Ernährungsbedarf eines Hundes oder einer Katze zu befriedigen, oder einem Leckerli, oder einem Snack.

21. Zusammensetzung nach Anspruch 20, wobei das Gellan auf der Oberfläche einer Komponente der Zusammensetzung ist.

22. Zusammensetzung nach Anspruch 19, wobei das Gellan etwa 0,1 bis 4 Gew.-% der Zusammensetzung ausmacht.

23. Zusammensetzung nach Anspruch 2, wobei das Gellan an einen Menschen mit einem Bedarf an den Antidurchfall-Wirkungen von Gellan verabreicht wird.

24. Zusammensetzung nach Anspruch 10, wobei das Gellan an einen Hund oder eine Katze mit einem Bedarf an den Antidurchfall-Wirkungen von Gellan verabreicht wird.

## Revendications

1. Composition comprenant de la gomme gellane destinée à être utilisée pour prévenir la diarrhée chez un mammifère qui a tendance à avoir la diarrhée ou pour réduire la diarrhée chez un mammifère qui présente déjà une diarrhée.

2. Composition selon la revendication 1, où le mammifère est un humain.

3. Composition selon la revendication 2, où la gomme gellane est administrée par voie orale.

4. Composition selon la revendication 3, où la gomme gellane est administrée sous la forme d'un liquide ou d'un solide.

5. Composition selon la revendication 4, où la gomme gellane est administrée en tant que solide sous la forme d'une gélule, d'un comprimé ou d'un cachet.

6. Composition selon la revendication 4, où la gomme gellane est administrée sous la forme d'un gel.

7. Composition selon la revendication 3, où la gomme gellane est administrée dans l'alimentation d'un humain.

8. Composition selon la revendication 7, où la gomme gellane est incorporée dans l'alimentation ou est présente sur la surface d'un composant alimentaire.

9. Composition selon la revendication 3, où la gomme gellane est administrée à une dose comprise entre environ 0,05 g/kg de poids corporel total et environ 2 g/kg de poids corporel total par jour.

10. Composition selon la revendication 1, où le mammifère est un chien ou un chat.

11. Composition selon la revendication 10, où la gomme gellane est administrée par voie orale.

12. Composition selon la revendication 11, où la gomme gellane est administrée sous la forme d'un liquide ou d'un solide.

13. Composition selon la revendication 12, où la gomme gellane est administrée en tant que solide sous la forme d'une gélule, d'un comprimé ou d'un cachet.

14. Composition selon la revendication 12, où la gomme gellane est administrée sous la forme d'un gel.

15. Composition selon la revendication 11, où la gomme gellane est administrée dans l'alimentation du chien ou du chat.

16. Composition selon la revendication 15, où la gomme gellane est incorporée dans l'alimentation ou est présente sur la surface d'un composant alimentaire.

17. Composition selon la revendication 11, où la gomme gellane est administrée à une dose comprise entre environ 0,05 g/kg de poids corporel total et environ 2 g/kg de poids corporel total par jour.

18. Composition selon l'une quelconque des revendications précédentes, qui est une forme posologique unitaire pharmaceutique appropriée pour une administration orale à un mammifère, comprenant une dose quotidienne ou une partie d'une dose quotidienne de gomme gellane suffisante pour prévenir la diarrhée chez un mammifère qui a tendance à avoir la diarrhée ou pour réduire la diarrhée chez un mammifère, en association avec un véhicule pharmaceutique.

19. Composition telle que définie dans l'une quelconque des revendications précédentes, qui est une composition alimentaire appropriée pour une ingestion par un chien ou un chat, et qui comporte au moins un composant alimentaire et une quantité de gomme gellane permettant de prévenir la diarrhée ou de réduire la diarrhée.

20. Composition selon la revendication 19, où l'aliment est sélectionné parmi une alimentation quotidienne suffisante pour répondre aux besoins nutritionnels d'un chien ou d'un chat, ou une friandise ou un en-cas.

21. Composition selon la revendication 20, où la gomme gellane est sur la surface d'un composant de la composition.

22. Composition selon la revendication 19, où la gomme gellane est comprise entre environ 0,1 et environ 4 % du poids total de la composition.

23. Composition selon la revendication 2, moyennant quoi la gomme gellane est administrée à un humain ayant besoin des effets antidiarrhéiques de la gellane.

24. Composition selon la revendication 10, moyennant quoi la gomme gellane est administrée à un chien ou à un chat ayant besoin des effets antidiarrhéiques de la gellane.
